# EUROPÄISCHE PATENTSCHRIFT

(11) **EP 1 140 755 B1**
(45) Veröffentlichungstag und Bekanntmachung des Hinweises auf die Patenterteilung: **28.04.2004**
(21) Anmeldenummer: 99959411.2
(22) Anmeldetag: 14.12.1999
(51) Int. Cl.: C07C 39/16, C07C 37/74

(54) **VERFAHREN ZUR HERSTELLUNG VON BISPHENOL A**
METHOD FOR PRODUCING BISPHENOL-A
PRODUCTION DE BISPHENOL A

(30) Priorität: 24.12.1998 DE 19860144
(43) Veröffentlichungstag der Anmeldung: 10.10.2001
(73) Patentinhaber: Bayer MaterialScience AG, 51368 Leverkusen (DE)
(72) Erfinder: LANZE, Rolf, D-47804 Krefeld (DE); NEUMANN, Rainer, D-47803 Krefeld (DE); KÜHLING, Steffen, B-9100 Sint Niklaas (BE); HEYDENREICH, Frieder, D-40593 Düsseldorf (DE); VAN OSSELAER, Tony, D-47800 Krefeld (DE)
(86) Internationale Anmeldenummer: PCT/EP1999/009917
(87) Internationale Veröffentlichungsnummer: WO 2000/039060

(56) Entgegenhaltungen:
- US-A- 5 629 457

## Beschreibung

Die vorliegende Erfindung betrifft ein Verfahren zur Herstellung von farbhellem Bisphenol A mit geringen Restgehalten an Sauerstoff und Phenol aus Mischkristallen von Bisphenol A und Phenol.

Aus EP-A 523 931 geht ein Verfahren hervor, bei dem ein Addukt von Bisphenol A und Phenol in einer Atmosphäre aufgeschmolzen wird, die max. 50 ppm, bevorzugt max. 10 ppm Sauerstoff enthält. Das Aufschmelzen wird bei 115-180°C, bevorzugt 120-150°C durchgeführt, unter einem Druck von 1,0 bis 5,0 atm, bevorzugt 1,0 bis 1,9 atm. Aus der Schmelze wird Phenol durch Verdampfen entfernt. Die Temperatur beträgt 160 bis 200°C, bevorzugt 170 bis 185°C, der Druck beträgt maximal 100 Torr (133 mbar), bevorzugt 5-40 Torr (7-53 mbar). Bevorzugt wird zuerst der Großteil des Phenols bei 160-185°C bei 20-80 mbar bis zu einem Restgehalt von 1-5 Gew.-% in einem Fallfilmverdampfer abdestilliert und anschließend das Restphenol mit Wasserdampf bei maximal 20 mbar und 170-185°C entfernt. Das Verhältnis Dampf:BPA beträgt 1:50 bis 1:5, bevorzugt 1:25 bis 1:10. Um die Abtrennung des Phenols vom BPA möglichst unter Ausschluß von Sauerstoff stattfinden zu lassen, wird gelehrt, alle produktberührten Oberflächen der verwendeten Apparatur mit einem organischen Lösungsmittel, bevorzugt Phenol, von Sauerstoff zu befreien. Die besten nach der Lehre der EP-A 523 931 erhaltenen Bisphenol-Produkte weisen Farbzahlen (APHA) von 10 auf.

Es wurde nun ein Verfahren gefunden, mit dem sich Produkte mit noch besseren Farbzahlen erhalten lassen, und das zudem den aufwendigen Waschschritt zur Entfernung von Sauerstoffspuren aus der Apparatur verzichtbar macht.

Gegenstand der Erfindung ist ein Verfahren zur Herstellung von Bisphenol A, bei dem ein Mischkristallisat aus Bisphenol A und Phenol bei Temperaturen von 100 bis 120°C aufgeschmolzen wird, die Schmelze anschließend mit Stickstoff inertisiert und am Kopf einer ersten Destillationseinheit kontinuierlich eingespeist wird, in der bei Temperaturen von 120 bis <160°C, bevorzugt 120 bis 157°C und Drücken von >80 bis 200 mbar, bevorzugt 85 bis 200 mbar, besonders bevorzugt 85 bis 120 mbar der Gehalt an Phenol in der Schmelze auf 10 bis 25 Gew.-% verringert wird, wobei gleichzeitig über den Sumpf der Destillationseinheit 0,5 bis 50 Vol.-%, bevorzugt 2 bis 20 Vol.-% Stickstoff, bezogen auf das Volumen an eingespeister Schmelze, zugeführt werden, und die von Sauerstoff befreite aufkonzentrierte Schmelze anschließend bei Temperaturen von 180 bis 220°C und 1 bis 1,5 bar Stickstoffpartialdruck von restlichem Phenol befreit wird.

Gegenstand der Erfindung ist außerdem ein Verfahren zur Herstellung von 75 bis 90 Gew.-% Bisphenol A und 10 bis 25 Gew.-% Phenol enthaltenden Schmelzen mit einem Sauerstoffgehalt < 1 ppm, bevorzugt < 100 ppb, besonders bevorzugt < 10 ppb, bei dem 60 Gew.-% Bisphenol A und 40 Gew.-% Phenol enthaltende Mischkristalle bei Temperaturen von 100 bis 120°C aufgeschmolzen werden, die Schmelze anschließend mit Stickstoff inertisiert und am Kopf einer Destillationseinheit kontinuierlich eingespeist wird, in der bei Temperaturen von 120 bis <160°C, bevorzugt 120 bis 157°C und Drücken von >80 bis 200 mbar, bevorzugt 85 bis 200 mbar, besonders bevorzugt 85 bis 120 mbar der Gehalt an Phenol in der Schmelze auf 10 bis 25 Gew.-% verringert wird, wobei gleichzeitig über den Sumpf der Destillationseinheit 0,5 bis 50 Vol.-% Stickstoff, bevorzugt 2 bis 20 Vol.-% Stickstoff, bezogen auf das Volumen an eingespeister Schmelze, zugeführt werden.

Im erfindungsgemäßen Verfahren werden als Edukt Mischkristallisate von Bisphenol A und Phenol eingesetzt, bevorzugt solche, die einen Bisphenolgehalt von 60 Gew.-% und einen Phenolgehalt von 40 Gew.-% aufweisen. Solche Mischkristallisate werden beispielsweise erhalten bei der sauer katalysierten Umsetzung von Phenol mit Aceton, wie sie z.B. in US-A 2,775,620 oder EP-A 342 758 beschrieben ist. In der Regel wird die Herstellung als kontinuierlich betriebener Prozeß durchgeführt. Das als Produkt anfallende Mischkristallisat von Bisphenol A und Phenol wird durch Filtration von der Mutterlauge abgetrennt, beispielsweise durch ein Drehfilter. Anschließend kann der Filterkuchen mit Phenol gewaschen werden, um anhaftende Verunreinigungen zu entfernen. Der resultierende Mischkristallkuchen kann als Edukt im erfindungsgemäßen Verfahren eingesetzt werden.

In einem ersten Schritt wird das Mischkristallisat bei Temperaturen von 100 bis 120°C aufgeschmolzen. Dies geschieht in einer Stickstoffatmosphäre. Aufgrund der geringen Reaktivität des Bisphenols gegenüber Sauerstoff bei diesen niedrigen Temperaturen ist es noch nicht erforderlich, auf strengsten Sauerstoffausschluß zu achten. Es können daher beim Aufschmelzen in der Atmosphäre Sauerstoffgehalte von 0,1 bis 2 Vol.-% toleriert werden. Es ist zweckmäßig, die Schmelze in einem mit Stickstoff inertisierten Behälter zu sammeln.

Die Schmelze, die noch gelösten Sauerstoff enthalten kann, wird anschließend am Kopf einer Destillationseinheit kontinuierlich eingespeist wird. Wichtig ist, daß in dieser Destilationseinheit eine möglichst große Austauschfläche erzeugt wird. Daher wird man bevorzugt eine Füllkörperkolonne, Packungskolonne, Bödenkolonne oder einen Fallfimverdampfer, Dünnschichtverdampfer oder Verdampfer mit Zwangsumlauf für diesen Schritt einsetzen.

In der Destillationseinheit wird bei Temperaturen von 120 bis <160°C, bevorzugt 120 bis 157°C und Drücken von >80 bis 200 mbar, bevorzugt 85 bis 200 mbar, besonders bevorzugt 85 bis 120 mbar der Gehalt an Phenol in der Schmelze auf 10 bis 25 Gew.-% verringert, wobei gleichzeitig über den Sumpf der Destillationseinheit 0,5 bis 50 Vol.-% Stickstoff, bevorzugt 2 bis 20 Vol.-% Stickstoff, bezogen auf das Volumen an eingespeister Schmelze, zugeführt werden.

Wichtig ist, daß bei diesem Schritt Schmelze und Stickstoff im Gegenstrom geführt werden, um in der Schmelze gelösten Sauerstoff möglichst vollständig auszutragen. Die Entfernung des Sauerstoffs wird hierbei durch das Verdampfen von Phenol und den verringerten Druck unterstützt. Der Stickstoffstrom unterstützt unter den angegebenen Bedingungen die Sauerstoffentfemung, ohne den Partialdruck von Phenol zu verringern. In der ersten Destillationseinheit wird unter schonenden Bedingungen gelöster Sauerstoff aus der Schmelze bis auf einen Restgehalt < 1 ppm entfernt. Durch optimale Wahl der Parameter lassen sich Schmelzen mit Sauerstoffgehalten < 100 ppb, ja sogar < 10 ppb erhalten. Auch treten unter den in diesem Schritt eingestellten Druck- und Temperaturbedingungen keine Verluste an Bisphenol A auf, die einen zusätzlichen Aufarbeitungsschritt erforderlich machen würden, bei dem Bisphenol A aus der Gasphase zurückgewonnen wird.

Die so erhaltene Schmelze kann anschließend in einem weiteren Schritt bei Temperaturen von 180 bis 220°C, bevorzugt 185 bis 195°C und Stickstoffpartialdrücken von 1 bis 1,5 bar, bevorzugt 1 bis 1,1 bar von restlichem Phenol befreit werden. Dabei wird durch die Schmelze ein Stickstoffstrom geleitet, um die Entfernung des Phenols zu unterstützen (,Strippen'). Das Verhältnis von Gas zu Schmelze liegt dabei bevorzugt bei 10 bis 1000 m³ Stickstoff pro t Schmelze. Die für die Phenolentfernung zu verwendenden Aggregate sind dem Fachmann geläufig; beispielsweise kann hierfür eine gepackte, geflutet betriebene Kolonne eingesetzt werden.

Die Phenolentfernung bei atmosphärischem Druck ermöglicht eine bessere Inertisierung der Schmelze als dies bei der Desorption unter Vakuumbedingungen gemäß EP-A 523 931 möglich ist und bietet zudem den Vorteil, daß bei Leckagen kein Sauerstoff in die Apparatur einströmen kann. Außerdem wird die Apparatur durch den Stickstoffstrom inertisiert und eventuell an den produktberührten Oberflächen adsorbierter Sauerstoff wird verdrängt, was ein Waschen dieser Oberflächen mit organischen Lösemittel überflüssig macht. Das so erhaltene Bisphenol A weist Phenolrestgehalte von maximal 50 ppm und Farbzahlen (gemessen nach ASTM D 1686) von weniger als 10 auf.

Die Schmelze kann anschließend direkt zu Prills verarbeitet oder zur Herstellung von Natriumbisphenolatlösungen für die Polycarbonatproduktion eingesetzt werden. Aus dem nach dem erfindungsgemäßen Verfahren erhaltenen Bisphenol A hergestellte Polycarbonate weisen einen kleineren Yellowness-Index (Y.I.) auf als Produkte des Standes der Technik. Das nach dem erfindungsgemäßen Verfahren erhaltene Bisphenol A läßt sich außerdem vorteilhaft zur Herstellung von Epoxidharzen verwenden.

### Beispiele

### Beispiel 1

Eine 60 Gew.-% Bisphenol A und 40 Gew.-% Phenol enthaltende Schmelze, in der noch Sauerstoff gelöst ist, wird in einer Menge von 1t/h kontinuierlich am Kopf einer Packungskolonne aufgegeben. Die Sumpftemperatur beträgt 145°C, der Druck am Kopf der Kolonne 100 mbar. Dem Fallfilmverdampfer der Kolonne werden im Gegenstrom zum Produktstrom kontinuierlich 50 l Stickstoff pro Stunde zugeführt. Das aus der Kolonne ausgespeiste Gemisch (75 Gew.-% Bisphenol A, 25 Gew.-% Phenol) wird in einem nachfolgenden Desorptionsschritt bei einer Schmelzetemperatur von 190°C mittels 225 m³ Stickstoff pro Stunde von Phenol befreit. Das im Stickstoffkreislauf enthaltende Phenol wird kondensiert. Pro Stunde werden 750 l des im Kreis geführten Stickstoffs ausgetauscht. Der Sauerstoffgehalt ini Stickstofikreislauf beträgt 0,4 ppm. Es wird eine phenolarme (50 ppm) BPA-Schmelze erhalten, die eine Farbzahl von 8 Hazen (gemessen nach ASTM D 1686) aufweist.

### Vergleichsbeispiel 1

Das Verfahren wird analog Beispiel 1 durchgeführt, jedoch wird auf die kontinuierliche Zufuhr von Stickstoff am Fallfilmverdampfer verzichtet. Der Sauerstoffgehalt im Stickstoffkreislauf beträgt 5 ppm. Es wird eine phenolarme (50 ppm) BPA-Schmelze erhalten, die eine Farbzahl von 12 Hazen aufweist.

### Vergleichsbeispiel 2

Eine 60 Gew.-% Bisphenol A und 40 Gew.-% Phenol enthaltende Schmelze, in der noch Sauerstoff gelöst ist, wird in einer Menge von 1t/h kontinuierlich direkt in die Desorption gefahren und bei einer Schmelzetemperatur von 190°C mittels 225 m³ Stickstoff pro Stunde von Phenol befreit. Das im Stickstoffkreislauf enthaltende Phenol wird kondensiert. Pro Stunde werden 750 l des im Kreis geführten Stickstoffs ausgetauscht. Der Sauerstoffgehalt im Stickstoffkreislauf beträgt 5 ppm. Es wird eine BPA-Schmelze mit einem Phenolgehalt von 250 ppm erhalten, die eine Farbzahl von 14 Hazen aufweist.

### Vergleichsbeispiel 3

Das Verfahren wird analog Vergleichsbeispiel 2 durchgeführt, jedoch wird die Phenoldesorption zur weiteren Absenkung des Phenolgehalts bei einer Schmelzetemperatur von 200°C betrieben. Es wird eine phenolarme (50 ppm) BPA-Schmelze erhalten, die eine Farbzahl von 18 Hazen aufweist.

## Patentansprüche

1. Verfahren zur Herstellung von Bisphenol A, bei dem ein Addukt aus Bisphenol A und Phenol bei Temperaturen von 100 bis 120°C aufgeschmolzen wird, die Schmelze anschließend mit Stickstoff inertisiert und am Kopf einer ersten Destillationseinheit kontinuierlich eingespeist wird, in der bei Temperaturen von 120 bis <160°C und Drücken von >80 bis 200 mbar der Gehalt an Phenol in der Schmelze auf 10 bis 25 Gew.-% verringert wird, wobei gleichzeitig über den Sumpf der Destillationseinheit 0,5 bis 50 Vol.-% Stickstoff, bezogen auf das Volumen an eingespeister Schmelze, zugeführt werden, und die von Sauerstoff befreite aufkonzentrierte Schmelze anschließend bei Temperaturen von 180 bis 220°C und 1 bis 1,5 bar Stickstoffpartialdruck von restlichem Phenol befreit wird, und wobei das Aufschmelzen in einer Stickstoffatmospähre geschieht, die maximal 2 Vol.-% Sauerstoff enthält.

2. Verfahren zur Herstellung von 75 bis 90 Gew.-% Bisphenol A und 10 bis 25 Gew.-% Phenol enthaltenden Schmelzen mit einem Sauerstoffgehalt < 1 ppm, bei dem 60 Gew.-% Bisphenol A und 40 Gew.-% Phenol enthaltende Mischkristalle bei Temperaturen von 100 bis 120°C aufgeschmolzen werden, die Schmelze anschließend mit Stickstoff inertisiert und am Kopf einer Destillationseinheit kontinuierlich eingespeist wird, in der bei Temperaturen von 120 bis <160°C und Drücken von >80 bis 200 mbar der Gehalt an Phenol in der Schmelze auf 10 bis 25 Gew.-% verringert wird, wobei gleichzeitig über den Sumpf der Destillationseinheit 0,5 bis 50 Vol.-% Stickstoff, bezogen auf das Volumen an eingespeister Schmelze, zugeführt werden, und wobei das Aufschmelzen in einer Stickstoffatmospähre geschieht, die maximal 2 Vol.-% Sauerstoff enthält.

## Claims

1. Process for the preparation of bisphenol A, wherein an adduct of bisphenol A and phenol is melted at temperatures of 100°C to 120°C, the melt is then rendered inert with nitrogen and fed in continuously at the top of a first distillation unit wherein, at temperatures of 120°C to <160°C and at pressures of >80 to 200 mbar, the phenol content of the melt is decreased to 10 to 25 wt.%, and at the same time 0.5 to 50 vol.% of nitrogen, based on the volume of introduced melt, is passed in via the bottom of the distillation unit, and the concentrated melt freed from oxygen is subsequently freed from residual phenol at temperatures of 180°C to 220°C and at 1 to 1.5 bar nitrogen partial pressure, and wherein the melting process is carried out in a nitrogen atmosphere containing at most 2 vol.% of oxygen.

2. Process for the preparation of melts containing 75 to 90 wt.% bisphenol A and 10 to 25 wt.% phenol and having an oxygen content of <1 ppm, wherein mixed crystals containing 60 wt.% bisphenol A and 40 wt.% phenol are melted at temperatures of 100°C to 120°C, the melt is then rendered inert with nitrogen and fed in continuously at the top of a distillation unit wherein, at temperatures of 120°C to <160°C and at pressures of >80 to 200 mbar, the phenol content of the melt is decreased to 10 to 25 wt.%, and at the same time 0.5 to 50 vol.% nitrogen, based on the volume of introduced melt, is passed in via the bottom of the distillation unit, and wherein the melting process is carried out in a nitrogen atmosphere containing at most 2 vol.% of oxygen.

## Revendications

1. Procédé de préparation de bisphénol A dans lequel un adduit de bisphénol A et de phénol est fondu à des températures de 100 à 120°C, la masse fondue est ensuite inertisée à l'azote et alimentée en continu à la tête d'une première unité de distillation dans laquelle le taux de phénol dans la masse fondue est réduit à 10 à 25% en poids à des températures de 120 à <160°C et des pressions de >80 à 200 mbars, 0,5 à 50 % en vol. d'azote par rapport au volume de la masse fondue amenée sont amenés simultanément au fond de l'unité de distillation et la masse fondue concentrée libérée de l'oxygène est ensuite libérée du phénol résiduel à des températures de 180 à 220°C et une pression partielle de l'azote de 1 à 1,5 bar, la fusion intervenant dans une atmosphère d'azote qui contient maximum 2% en vol. d'oxygène.

2. Procédé de préparation de masses fondues contenant 75 à 90% en poids de bisphénol A et 10 à 25% en poids de phénol avec un taux d'oxygène <1 ppm, dans lequel des cristaux mixtes contenant 60% en poids de bisphénol A et 40% en poids de phénol sont fondus à des températures de 100 à 120°C, la masse fondue est ensuite inertisée à l'azote et alimentée en continu à la tête d'une unité de distillation dans laquelle le taux de phénol dans la masse fondue est réduit à 10 à 25% en poids à des températures de 120 à <160°C et des pressions de >80 à 200 mbars, 0,5 à 50% en vol. d'azote par rapport au volume de la masse fondue amenée étant amenés simultanément sur le fond de l'unité de distillation et la masse fondue intervenant dans une atmosphère d'azote qui contient maximum 2% en vol. d'oxygène.
